# EUROPÄISCHE PATENTSCHRIFT

(11) **EP 1 474 406 B1**
(45) Veröffentlichungstag und Bekanntmachung des Hinweises auf die Patenterteilung: **21.03.2007**
(21) Anmeldenummer: 03701536.9
(22) Anmeldetag: 22.01.2003
(51) Int. Cl.: C07D 277/56, A01N 43/78, C07F 5/02

(54) **DISUBSTITUIERTE THIAZOLYLCARBOXANILIDE UND IHRE VERWENDUNG ALS MIKROBIZIDE**
DISUBSTITUTED THIAZOLYL CARBOXANILIDES AND THEIR USE AS MICROBICIDES
THIAZOLYLCARBOXANILIDES DISUBSTITUES ET LEUR UTILISATION COMME MICROBICIDES

(30) Priorität: 04.02.2002 DE 10204390
(43) Veröffentlichungstag der Anmeldung: 10.11.2004
(73) Patentinhaber: Bayer CropScience Aktiengesellschaft, 40789 Monheim (DE)
(72) Erfinder: DUNKEL, Ralf, 40789 Monheim (DE); ELBE, Hans-Ludwig, 42329 Wuppertal (DE); RIECK, Heiko, F-69110 Ste. Foy-lès-Lyon (FR); KUCK, Karl-Heinz, 40764 Langenfeld (DE); WACHENDORFF-NEUMANN, Ulrike, 56566 Neuwied (DE); MAULER-MACHNIK, Astrid, 42799 Leichlingen (DE)
(86) Internationale Anmeldenummer: PCT/EP2003/000588
(87) Internationale Veröffentlichungsnummer: WO 2003/066609

(56) Entgegenhaltungen:
- EP-A- 0 371 950
- EP-A- 0 545 099
- WO-A-02/08197
- DATABASE CAPLUS [Online] CHEMICAL ABSTRACTS SERVICE, COLUMBUS, OHIO, US; retrieved from STN Database accession no. 1995:784957 XP002238293 & JP 07 145156 A (MITSUI TOATSU CHEMICALS) 6. Juni 1995 (1995-06-06)

## Beschreibung

Die vorliegende Erfindung betrifft neue Thiazolylcarboxanilide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxanilide fungizide Eigenschaften besitzen (vergleiche z.B. EP 0 545 099 und WO 02/08197). Die Wirksamkeit der dort beschriebenen Stoffe ist gut, lässt aber bei niedrigen Aufwandmengen in manchen Fällen zu wünschen übrig.

Es wurden nun neue Thiazolylcarboxanilide der Formel (I) 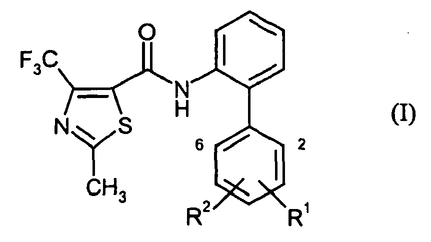 in welcher
- R¹ und R²: unabhängig voneinander für Halogen, Cyano, Nitro, C₁-C₆-Alkyl, C₂-C₆-Alkenyl, C₁-C₄-Alkoxy, C₁-C₄-Alkylthio, C₁-C₄-Alkylsulfonyl, C₃-C₆-Cycloalkyl, oder für C₁-C₄-Halogenalkyl, C₁-C₄-Halogenalkoxy, C₁-C₄-Halogenalkylthio oder C₁-C₄-Halogenalkylsulfonyl mit jeweils 1 bis 5 Halogenatomen stehen,
- R¹ und R²: außerdem, wenn sie in ortho-Position zueinander stehen, gemeinsam für gegebenenfalls durch Halogen oder C₁-C₆-Alkyl substituiertes Alkenylen stehen,
gefunden.

Weiterhin wurde gefunden, dass man Thiazolylcarboxanilide der Formel (I) erhält, indem man
a) Thiazolcarbonsäurehalogenide der Formel (II) 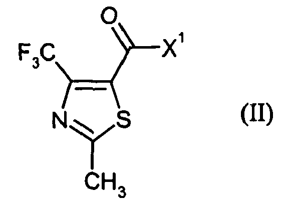 in welcher
   - X¹: für Halogen steht,
   mit Anilinderivaten der Formel (III) 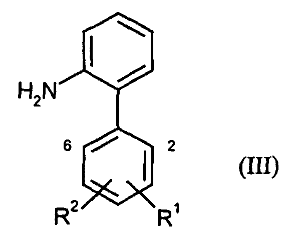 in welcher
   - R¹ und R²: die oben angegebenen Bedeutungen haben,
   gegebenenfalls in Gegenwart eines Säurebindemittels und gegebenenfalls in Gegenwart eines Verdünnungsmittels umsetzt, oder
b) Halogenthiazolcarboxanilide der Formel (IV) 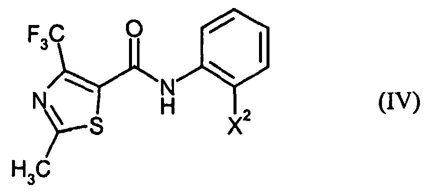 in welcher
   - X²: für Brom oder Iod steht,
   mit Boronsäurederivaten der Formel (V) 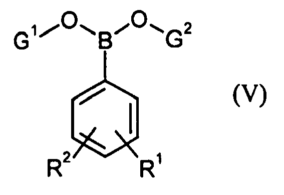 in welcher
   - R¹ und R²: die oben angegebenen Bedeutungen haben,
   - G¹ und G²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
c) Thiazolcarboxanilid-Boronsäure-Derivate der Formel (VI) 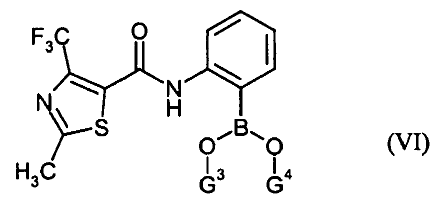 in welcher
   - G³ und G⁴: jeweils für Wasserstoff oder zusammen für Tetramethylethylen stehen,
   mit Halogenbenzolderivaten der Formel (VII) 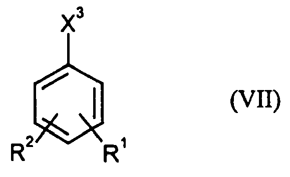 in welcher
   - R¹ und R²: die oben angegebenen Bedeutungen haben und

   - X³: für Brom, Iod oder Trifluormethylsulfonyloxy steht,
   in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die neuen Thiazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Thiazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Thiazolylcarboxanilide sind durch die Formel (I) allgemein definiert.

Bevorzugt sind Thiazolylcarboxanilide der Formel (I), in welcher
- R¹ und R²: unabhängig voneinander für Fluor, Chlor, Brom, Cyano, Nitro, Methyl, Ethyl, n- oder i-Propyl, n-, i-, s- oder t-Butyl, Methoxy, Ethoxy, Methylthio, Ethylthio, n- oder i-Propylthio, Cyclopropyl, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio stehen,
- R¹ und R²: außerdem, wenn sie in ortho-Position zueinander stehen, gemeinsam für gegebenenfalls durch Fluor, Chlor, Brom oder Methyl substituiertes Butadienylen stehen.

Besonders bevorzugt sind Thiazolylcarboxanilide der Formel (I), in welcher
- R¹ und R²: unabhängig voneinander für Fluor, Chlor, Brom, Methyl, Trifluormethyl, Difluormethoxy oder Trifluormethoxy stehen.

Besonders bevorzugt sind Thiazolylcarboxanilide der Formel (I), in welcher wenigstens einer der Reste R¹ und R² für Iod steht.

Ganz besonders bevorzugt sind Thiazolylcarboxanilide der Formel (I), in welcher R¹ für Fluor, Chlor, Brom oder Iod, insbesondere für Fluor oder Chlor steht.

Ganz besonders bevorzugt sind Thiazolylcarboxanilide der Formel (I), in welcher R² für Fluor, Chlor, Brom oder Iod steht.

Ganz besonders bevorzugt sind Thiazolylcarboxanilide der Formel (I), in welcher R¹ für Fluor und R² für Chlor steht.

Ganz besonders bevorzugt sind Thiazolylcarboxanilide der Formel (I), in welcher R¹ für Fluor und R² für Fluor steht.

Ganz besonders bevorzugt sind Thiazolylcarboxanilide der Formel (I), in welcher R¹ für Methyl oder Trifluormethyl steht.

Ganz besonders bevorzugt sind Thiazolylcarboxanilide der Formel (I), in welcher R² für Methyl oder Trifluormethyl steht.

Verwendet man 2-Methyl-4-(trifluormethyl)-1,3-thiazol-5-carbonylchlorid und 3'-Chlor-4'-fluor-1,1'-biphenyl-2-amin als Ausgangsstoffe sowie ein Base, so kann der Verlauf des erfindungsgemäßen Verfahrens a) durch folgende Reaktionsgleichung veranschaulicht werden:

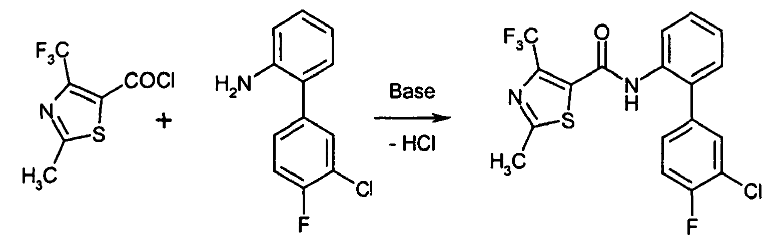

Die zur Durchführung des erfindungsgemäßen Verfahrens a) als Ausgangsstoffe benötigten Thiazolcarbonsäurehalogenide sind durch die Formel (II) allgemein definiert. In dieser Formel (II) steht X¹ bevorzugt für Chlor.

Die Thiazolcarbonsäurehalogenide der Formel (II) sind bekannt und/oder lassen sich nach bekannten Verfahren herstellen (vergleiche z.B. EP 0 276 177).

Die weiterhin zur Durchführung des erfindungsgemäßen Verfahrens a) als Ausgangsstoffe benötigten Aniline sind durch die Formel (III) allgemein definiert. In dieser Formel (III) stehen R¹ und R² bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden.

Die Anilin-Derivate der Formel (III) sind bekannt und/oder lassen sich nach bekannten Methoden herstellen (vgl. z.B. Bull. Korean Chem. Soc. 2000, 21, 165-166; Chem. Pharm. Bull. 1992, 40, 240-4; JP 09132567).

Verwendet man *N*-(2-Iodphenyl)-2-methyl-4-(trifluormethyl)-1,3-thiazol-5-carboxamid und 3-Chlor-4-fluorphenylboronsäure als Ausgangsstoffe sowie einen Katalystor und eine Base, so kann der Verlauf des erfindungsgemäßen Verfahrens b) kann durch folgende Reaktionsgleichung veranschaulicht werden:

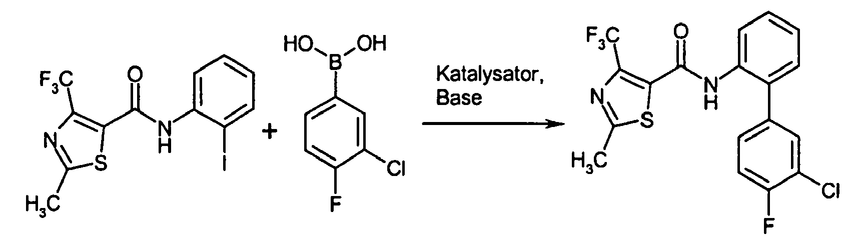

Die zur Durchführung des erfindungsgemäßen Verfahrens b) als Ausgangsstoffe benötigten Halogenthiazolcarboxanilide sind durch die Formel (IV) allgemein definiert. In dieser Formel (IV) steht X² bevorzugt für Brom oder Iod.

Die Halogenthiazolcarboxanilide der Formel (IV) werden erhalten, indem man
d) Thiazolcarbonsäurehalogenide der Formel (II) 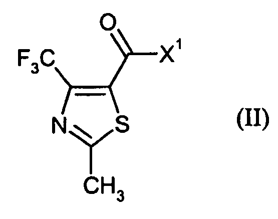 in welcher
   - X¹: für Halogen steht,
   mit 2-Bromanilin oder 2-Iodanilin umsetzt.

Die zur Durchführung des Verfahrens d) als Ausgangsstoffe benötigten Thiazolcarbonsäurehalogenide der Formel (II) sind bereits weiter oben im Zusammenhang mit dem erfindungsgemäßen Verfahren a) beschrieben worden.

Die weiterhin zur Durchführung des Verfahrens d) als Ausgangsstoffe benötigten Stoffe 2-Bromanilin oder 2-Iodanilin sind bekannte Synthesechemikalien.

Die weiterhin zur Durchführung des erfindungsgemäßen Verfahrens b) als Ausgangsstoffe benötigten Boronsäuren sind durch die Formel (V) allgemein definiert. In dieser Formel (V) stehen R¹ und R² bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für R¹ und R² angegeben wurden. G¹ und G² stehen bevorzgut jeweils für Wasserstoff oder zusammen für Tetramethylethylen.

Boronsäuren der Formel (V) sind bekannte Synthesechemikalien. Sie können auch unmittelbar vor der Reaktion direkt aus Halogenbenzolderivaten und Boronsäureestem hergestellt und ohne Aufarbeitung weiter umgesetzt werden (siehe auch die Herstellungsbeispiele).

Verwendet man 2-Methyl-*N*-[2-(4,4,5,5-tetramethyl-1,3,2-dioxaborolan-2-yl)phenyl]-4-(trifluormethyl)-1,3-thiazol-5-carboxamid und 3-Chlor-4-fluorphenyltrifluormethansulfonsäure als Ausgangsstoffe sowie einen Katalystor und eine Base, so kann der Verlauf des erfindungsgemäßen Verfahrens c) kann durch folgende Reaktionsgleichung veranschaulicht werden:

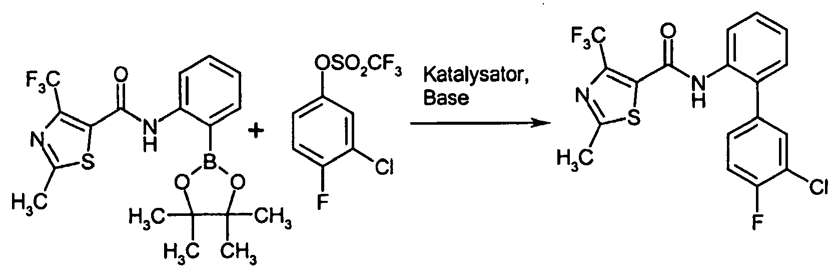

Die zur Durchführung des erfindungsgemäßen Verfahrens c) als Ausgangsstoffe benötigten Thiazolcarboxanilid-Boronsäure-Derivate sind durch die Formel (VI) allgemein definiert. In dieser Formel (VI) stehen G³ und G⁴ bevorzugt jeweils für Wasserstoff oder zusammen für Tetramethylethylen.

Die Thiazolcarboxanilid-Boronsäure-Derivate der Formel (VI) werden erhalten, indem man
e) Thiazolcarbonsäurehalogenide der Formel (II) 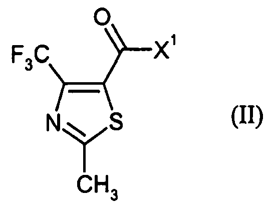 in welcher
   - X¹: für Halogen steht,
   mit Anilinboronsäurederivaten der Formel (VIII) 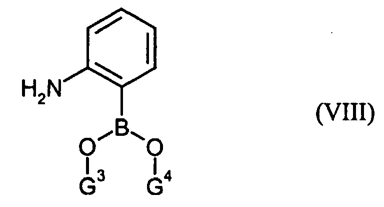 in welcher
   - G³ und G⁴: die oben angegebenen Bedeutungen haben,
   gegebenenfalls in Gegenwart eines Säurebindemittels und gegebenenfalls in Gegenwart eines Verdünnungsmittels umsetzt.

Die zur Durchführung des Verfahrens e) als Ausgangsstoffe benötigten Thiazolcarbonsäurehalogenide der Formel (II) sind bereits weiter oben im Zusammenhang mit dem erfindungsgemäßen Verfahren a) beschrieben worden.

Die weiterhin zur Durchführung des Verfahrens e) als Ausgangsstoffe benötigten Anilinboronsäurederivate sind durch die Formel (VIII) allgemein definiert. In dieser Formel (VIII) stehen G³ und G⁴ bevorzugt jeweils für Wasserstoff oder zusammen für Tetramethylethylen.

Die zur Durchführung des Verfahrens e) als Ausgangsstoffe benötigten Anilinboronsäurederivate der Formel (VIII) sind bekannte Synthesechemikalien.

Die weiterhin zur Durchführung des erfindungsgemäßen Verfahrens c) als Ausgangsstoffe benötigten Halogenbenzolderivate sind durch die Formel (VII) allgemein definiert. In dieser Formel (VII) stehen R¹ und R² bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für R¹ und R² angegeben wurden. X³ steht bevorzugt für Brom, Iod oder Trifluormethylsulfonyloxy.

Als Verdünnungsmittel zur Durchführung der Verfahren a), d) und e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die Verfahren a), d) und e) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der Verfahren a), d) und e) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens a) zur Herstellung der Verbindungen der Formel (I) setzt man pro Mol des Thiazolcarbonsäurehalogenides der Formel (II) im allgemeinen 0,2 bis 5 Mol, vorzugsweise 0,5 bis 2 Mol an Anilinderivat der Formel (III) ein. Die Aufarbeitung erfolgt nach üblichen Methoden.

Zur Durchführung des Verfahrens d) zur Herstellung der Verbindungen der Formel (III) setzt man pro Mol des Thiazolcarbonsäurehalogenides der Formel (II) im allgemeinen 0,2 bis 5 Mol, vorzugsweise 0,5 bis 2 Mol an o-Brom- oder o-Iodanilin ein. Die Aufarbeitung erfolgt nach üblichen Methoden.

Zur Durchführung des Verfahrens e) zur Herstellung der Verbindungen der Formel (VI) setzt man pro Mol des Thiazolcarbonsäurehalogenides der Formel (II) im allgemeinen 0,2 bis 5 Mol, vorzugsweise 0,5 bis 2 Mol an Anilinboronsäurederivat der Formel (VIII) ein. Die Aufarbeitung erfolgt nach üblichen Methoden.

Als Verdünnungsmittel zur Durchführung der erfindungsgemäßen Verfahren b) und c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahrens b) und c) in einem größeren Bereich variiert werden. Im allgemeinen arbeitet man bei Temperaturen von 0°C bis 150°C, vorzugsweise bei Temperaturen von 20°C bis 110°C.

Die erfindungsgemäßen Verfahren b) und c) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluaorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b) und c) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder (1,1'-Bis(diphenylphosphino)ferrocenpalladium(II)chlorid) infrage.

Es kann auch ein Palladiumkomplex in der Reaktionsmischung erzeugt werden, wenn man ein Palladiumsalz und ein Komplexligand, wie beispielsweise Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-tolyl)-phosphan, Natrium-3-(Diphenylphosphino)benzolsulfonat, Tris-2-(methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens b) zur Herstellung der Verbindungen der Formel (I) setzt man pro Mol des Halogenthiazolcarboxanilids der Formel (IV) im allgemeinen 1 bis 15 Mol, vorzugsweise 2 bis 8 Mol an Boronsäure der Formel (V) ein. Die Aufarbeitung erfolgt nach üblichen Methoden.

Zur Durchführung des erfindungsgemäßen Verfahrens zur Herstellung der Verbindungen der Formel (I) setzt man pro Mol des Thiazolcarboxanilid-Boronsäure-Derivate der Formel (VI) im allgemeinen 1 bis 15 Mol, vorzugsweise 2 bis 8 Mol an Halogenbenzolderivat der Formel (VII) ein. Die Aufarbeitung erfolgt nach üblichen Methoden.

Die Verfahren a), b), c) und d) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflarizenstärkenden (resistenzinduzierenden) Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokolation mit unerwünschten Mikroorganismen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen. Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, kömige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
Fungizide:
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenhexamid, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin, Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Iprovalicarb, Irumamycin, Isoprothiolan, Isovaledione,
   Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
   Quinconazol, Quintozen (PCNB), Quinoxyfen
   Schwefel und Schwefel-Zubereitungen, Spiroxamine
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol, Validamycin A, Vinclozolin, Viniconazol,
   Zarilamid, Zineb, Ziram sowie
      Dagger G, OK-8705, OK-8801,
      α-(1,1-Dimethylethyl)-β-(2-phenoxyethyl)-1H-1,2,4-triazol-1-ethanol,
      α-(2,4-Dichlorphenyl)-β-fluor-β-propyl-1H-1,2,4-triazol-1-ethanol,
      α-(2,4-Dichlorphenyl)-β-methoxy-α-methyl-1H-1,2,4-triazol-1-ethanol,
      α-(5-Methyl-1,3-dioxan-5-yl)-β-[[4-(trifluormethyl)-phenyl]-methylen]-1H-1,2,4-triazol-1-ethanol,
      (5RS,6RS)-6-Hydroxy-2,2,7,7-tetramethyl-5-(1H-1,2,4-triazol-1-yl)-3-octanon,
      (E)-a-(Methoxyimino)-N-methyl-2-phenoxy-phenylacetamid,
      1-(2,4-Dichlorphenyl)-2-(1H-1,2,4-triazol-1-yl)-ethanon-O-(phenylmethyl)-oxim,
      1-(2-Methyl-1-naphthalenyl)-1H-pyrrol-2,5-dion,
      1-(3,5-Dichlorphenyl)-3-(2-propenyl)-2,5-pyrrolidindion,
      1-[(Diiodmethyl)-sulfonyl]-4-methyl-benzol,
      1-[[2-(2,4-Dichlorphenyl)-1,3-dioxolan-2-yl]-methyl]-1H-imidazol,
      1-[[2-(4-Chlorphenyl)-3-phenyloxiranyl]-methyl]-1H-1,2,4-triazol,
      1-[1-[2-[(2,4-Dichlorphenyl)-methoxy]-phenyl]-ethenyl]-1H-imidazol,
      1-Methyl-5-nonyl-2-(phenylmethyl)-3-pyrrolidinol,
      2',6'-Dibrom-2-methyl-4'-trifluormethoxy-4'-trifluor-methyl-1,3-thiazol-5-carboxanilid,
      2,6-Dichlor-5-(methylthio)-4-pyrimidinyl-thiocyanat,
      2,6-Dichlor-N-(4-trifluormethylbenzyl)-benzamid,
      2,6-Dichlor-N-[[4-(trifluormethyl)-phenyl]-methyl]-benzamid,
      2-(2,3,3-Triiod-2-propenyl)-2H-tetrazol,
      2-[(1-Methylethyl)-sulfonyl]-5-(trichlormethyl)-1,3,4-thiadiazol,
      2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
      2-Aminobutan,
      2-Brom-2-(brommethyl)-pentandinitril,
      2-Chlor-N-(2,3-dihydro-1,1,3-trimethyl-1H-inden-4-yl)-3-pyridincarboxamid,
      2-Chlor-N-(2,6-dimethylphenyl)-N-(isothiocyanatomethyl)-acetamid,
      2-Phenylphenol(OPP),
      3,4-Dichlor-1-[4-(difluormethoxy)-phenyl]-1H-pyrrol-2,5-dion,
      3,5-Dichlor-N-[cyan[(1-methyl-2-propynyl)-oxy]-methyl]-benzamid,
      3-(1,1-Dimethylpropyl-1-oxo-1H-inden-2-carbonitril,
      3-[2-(4-Chlorphenyl)-5-ethoxy-3-isoxazolidinyl]-pyridin,
      4-Chlor-2-cyan-N,N-dimethyl-5-(4-methylphenyl)-1H-imidazol-1-sulfonamid,
      4-Methyl-tetrazolo[1,5-a]quinazolin-5(4H)-on,
      8-Hydroxychinolinsulfat,
      9H-Xanthen-9-carbonsäure-2-[(phenylamino)-carbonyl]-hydrazid,
      bis-(1-Methylethyl)-3-methyl-4-[(3-methylbenzoyl)-oxy]-2,5-thiophendicarboxylat,
      cis-1-(4-Chlorphenyl)-2-(1H-1,2,4-triazol-1-yl)-cycloheptanol,
      cis-4-[3-[4-(1,1-Dimethylpropyl)-phenyl-2-methylpropyl]-2,6-dimethyl-morpholinhydrochlorid,
      Ethyl-[(4-chlorphenyl)-azo]-cyanoacetat,
      Kaliumhydrogencarbonat,
      Methantetrathiol-Natriumsalz,
      Methyl-1-(2,3-dihydro-2,2-dimethyl-1H-inden-1-yl)-1H-imidazol-5-carboxylat,
      Methyl-N-(2,6-dimethylphenyl)-N-(5-isoxazolylcarbonyl)-DL-alaninat,
      Methyl-N-(chloracetyl)-N-(2,6-dimethylphenyl)-DL-alaninat,
      N-(2,6-Dimethylphenyl)-2-methoxy-N-(tetrahydro-2-oxo-3-furanyl)-acetamid,
      N-(2,6-Dimethylphenyl)-2-methoxy-N-(tetrahydro-2-oxo-3-thienyl)-acetamid,
      N-(2-Chlor-4-nitrophenyl)-4-methyl-3-nitro-benzolsulfonamid,
      N-(4-Cyclohexylphenyl)-1,4,5,6-tetrahydro-2-pyrimidinamin,
      N-(4-Hexylphenyl)-1,4,5,6-tetrahydro-2-pyrimidinamin,
      N-(5-Chlor-2-methylphenyl)-2-methoxy-N-(2-oxo-3-oxazolidinyl)-acetamid,
      N-(6-Methoxy)-3-pyridinyl)-cyclopropancarboxamid,
      N-[2,2,2-Trichlor-1-[(chloracetyl)-amino]-ethyl]-benzamid,
      N-[3-Chlor-4,5-bis-(2-propinyloxy)-phenyl]-N'-methoxy-methanimidamid,
      N-Formyl-N-hydroxy-DL-alanin-Natriumsalz,
      O,O-Diethyl-[2-(dipropylamino)-2-oxoethyl]-ethylphosphoramidothioat,
      O-Methyl-S-phenyl-phenylpropylphosphoramidothioate,
      S-Methyl-1,2,3-benzothiadiazol-7-carbothioat,
      spiro[2H]-1-Benzopyran-2,1'(3'H)-isobenzofuran]-3'-on,
      4-[3,4-Dimethoxyphenyl)-3-(4-fluorphenyl)-acryloyl]-morpholin
Bakterizide:
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
Insektizide / Akarizide / Nematizide:
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Indoxacarb, Isazofos, Isofenphos, Isoxathion, Ivermectin,
   Kernpolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos, Ribavirin,
   Salithion, Sebufos, Silafluofen, Spinosad, Spirodiclofen, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
      YI 5302, Zeta-cypermethrin, Zolaprofos
      (1R-cis)-[5-(Phenylmethyl)-3-furanyl]-methyl-3-[(dihydro-2-oxo-3(2H)-furanyliden)-methyl]-2,2-dimethylcyclopropancarboxylat
      (3-Phenoxyphenyl)-methyl-2,2,3,3-tetramethylcyclopropanecarboxylat
      1-[(2-Chlor-5-thiazolyl)methyl]tetrahydro-3,5-dimethyl-N-nitro-1,3,5-triazin-2(1H)-imin
      2-(2-Chlor-6-fluorphenyl)-4-[4-(1,1-dimethylethyl)phenyl]-4,5-dihydro-oxazol
      2-(Acetlyoxy)-3-dodecyl-1,4-naphthalindion
      2-Chlor-N-[[[4-(1-phenylethoxy)-phenyl]-amino]-carbonyl]-benzamid
      2-Chlor-N-[[[4-(2,2-dichlor-1,1-difluorethoxy)-phenyl]-amino]-carbonyl]-benzamid
      3-Methylphenyl-propylcarbamat
      4-[4-(4-Ethoxyphenyl)-4-methylpentyl]-1-fluor-2-phenoxy-benzol
      4-Chlor-2-(1,1-dimethylethyl)-5-[[2-(2,6-dimethyl-4-phenoxyphenoxy)ethyl]thio]-3(2H)-pyridazinon
      4-Chlor-2-(2-chlor-2-methylpropyl)-5-[(6-iod-3-pyridinyl)methoxy]-3(2H)-pyridazinon
      4-Chlor-5-[(6-chlor-3-pyridinyl)methoxy]-2-(3,4-dichlorphenyl)-3(2H)-pyridazinon
      Bacillus thuringiensis strain EG-2348
      Benzoesäure [2-benzoyl-1-(1,1-dimethylethyl)-hydrazid
      Butansäure 2,2-dimethyl-3-(2,4-dichlorphenyl)-2-oxo-1-oxaspiro[4.5]dec-3-en-4-ylester
      [3-[(6-Chlor-3-pyridinyl)methyl]-2-thiazolidinyliden]-cyanamid
      Dihydro-2-(nitromethylen)-2H-1,3-thiazine-3(4H)-carboxaldehyd
      Ethyl-[2-[[1,6-dihydro-6-oxo-1-(phenylmethyl)-4-pyridazinyl]oxy]ethyl]-carbamat
      N-(3,4,4-Trifluor-1-oxo-3-butenyl)-glycin
      N-(4-Chlorphenyl)-3-[4-(difluonnethoxy)phenyl]-4,5-dihydro-4-phenyl-1H-pyrazol-1-carboxamid
      N-[(2-Chlor-5-thiazolyl)methyl]-N'-methyl-N"-nitro-guanidin
      N-Methyl-N'-(-methyl-2-propenyl)-1,2-hydrazindicarbothioamid
      N-Methyl-N'-2-propenyl-1,2-hydrazindicarbothioamid
      O,O-Diethyl-[2-(dipropylamino)-2-oxoethyl]-ethylphosphoramidothioat
      N-Cyanomethyl-4-trifluormethyl-nicotinamid
      3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha.

Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffinischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Herstellungsbeispiel

### Beispiel 1

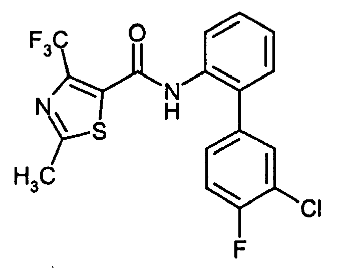

### Verfahren a)

0,29 g (1,3 mmol) 3'-Chlor-4'-fluor-1,1'-biphenyl-2-amin und 0,36 g (1,56 mmol) 2-Methyl-4-(trifluormethyl)-1,3-thiazol-5-carbonylchlorid werden in 6 ml Tetrahydrofuran gelöst und mit 0,36 ml (2,6 mmol) Triethylamin versetzt. Die Reaktionslösung wird für 16 h bei 60°C gerührt. Zur Aufarbeitung wird aufkonzentriert und mit Cyclohexan/Essigsäureethylester an Kieselgel chromatographiert.

Man erhält 0,52 g (95 % d. Th.) *N*-(3'-Chlor-4'-fluor-1,1'-biphenyl-2-yl)-2-methyl-4-(trifluormethyl)-1,3-thiazol-5-carboxamid mit dem logP (pH2,3) = 3,58.

### Beispiel 2

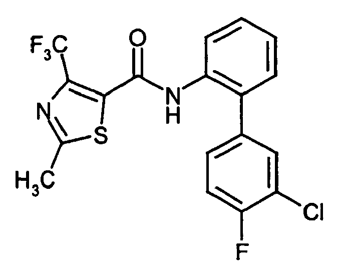

### Verfahren b)

0,185 g (0,88 mmol) 4-Brom-2-chlor-1-fluorbenzol, 0,243 g (2,5 mmol) Kaliumacetat und 0,21 g (0,83 mmol) Pinacoldiboronsäureester werden in 8 ml Dimethylsulfoxid suspendiert und unter Argon mit einer katalytischen Menge (ca. 5 mol%) 1,1'-Bis(diphenylphosphino)ferrocenpalladium(II)chlorid versetzt. Die Reaktionslösung wird 2 Stunden auf 80 °C erhitzt, dann erfolgt die Zugabe von 0,33 g (0,8 mmol) *N*-(2-Iodphenyl)-2-methyl-4-(trifluormethyl)-1,3-thiazol-5-carboxamid, 2,5 ml einer 2M Natriumcarbonatlösung, sowie einer weiteren katalytischen Menge an Bis(diphenylphosphino)ferrocenpalladium(II)chlorid. Für 16 h wird bei 80°C gerührt. Zur Aufarbeitung wird die Reaktionslösung in 50 ml Essigsäureethylester oder Dichlormethan aufgenommen und mit Wasser (5-10 ml) gewaschen, über Magnesiumsulfat getrocknet, mit Aktivkohle versetzt, filtriert und eingeengt. Der Rückstand wird mit Cyclohexan/Essigsäureethylester an Kieselgel chromatographiert.

Man erhält 0,18 g (54 % d. Th.) *N*-(3'-Clor-4'-fluor-1,1'-biphenyl-2-yl)-2-methyl-4-(trifluormethyl)-1,3-thiazol-5-carboxamid mit dem logP (pH2,3) = 3,60.

Analog den Beispielen 1 und 2, sowie entsprechend den Angaben in den allgemeinen Beschreibungen der Verfahren a) und b), werden die in der nachstehenden Tabelle 1 genannten Verbindungen der Formel (I) erhalten.

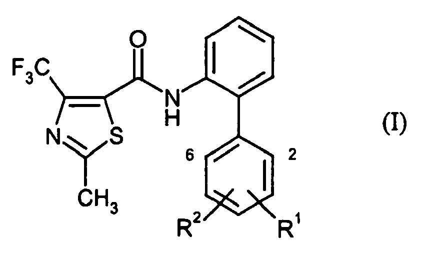

**Tabelle 1**

| **Bsp.** | **R¹** | **R²** | **logP** | **Fp./°C** |
|---|---|---|---|---|
| 3 | 3-CH=CH-CH=CH-4 | | 4,6 | |
| 4 | 3-F | 4-F | 3,31 | 120-122 |
| 5 | 3-F | 5-F | 3,35 | 133-134 |
| 6 | 2-F | 4-F | 3,21 | |
| 7 | 3-F | 4-Cl | 3,61 | 139-141 |
| 8 | 3-Cl | 4-Cl | 3,89 | |
| 9 | 3-CF₃ | 4-F | 3,76 | |
| 10 | 3-CH₃ | 4-Cl | 4,03 | |
| 11 | 3-CF₃ | 4-Cl | 4,03 | |
| 12 | 3-CF₃ | 4-CH₃ | 4,03 | |
| 13 | 3-CF₃ | 4-OCF₃ | 4,08 | |
| 14 | 3-Cl | 5-Cl | 3,63 | |
| 15 | 3-F | 4-OCF₃ | 3,94 | |
| 16 | 2-CH₃ | 4-Cl | 4,23 | |
| 17 | 2-Cl | 4-Cl | 3,86 | |
| 18 | 3-Cl | 4-CH₃ | | |
| 19 | 2-F | 4-Cl | 3,52 | |
| 20 | 3-F | 5-Cl | 3,67 | |
| 21 | 2-F | 4-Br | 3,63 | |
| 22 | 3-F | 4-Br | 3,68 | |
| 23 | 3-Cl | 4-Br | 3,98 | |
| 24 | 2-F | 4-I | 3,76 | |
| 25 | 3-F | 4-CF₃ | 3,75 | |

### Herstellung eines Vorproduktes der Formel (III)

### Beispiel (III-1)

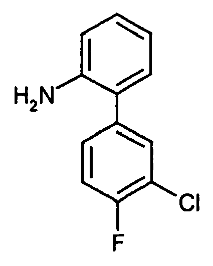

38,8 g (223 mmol) 3-Chlor-4-fluorphenylboronsäure, 40,6 g (186 mmol) 2-Iodanilin werden in 220 ml Toluol, 22 ml Ethanol und 45 ml einer 4 M Natriumhydrogencarbonatlösung unter Argon gelöst. Hierzu gibt man 4,3 g (4 mmol) Tetrakis(triphenylphosphin)palladium(0) lässt die Reaktionslösung 16 Stunden bei 80 °C unter Argon rühren. Die organische Phase wird abgetrennt, über Magnesiumsulfat getrocknet und aufkonzentriert. Der Rückstand wird mit Cyclohexan/Essigsäureethylester an Kieselgel chromatographiert.

Man erhält 22,5 g (48 % d. Th.) 3'-Chlor-4'-fluor-1,1'-biphenyl-2-amin (Reinheit 88%) mit dem logP (pH2,3) = 3,01.

### Herstellung der Vorprodukte der Formel (IV)

### Beispiel (IV-1)

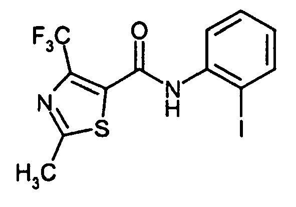

10,2 g (46 mmol) 2-Iodanilin werden in 100 ml Acetonitril gelöst und mit 9,52 g (69 mmol) Kaliumcarbonat versetzt. Nach Zugabe von 10,7 g (46 mmol) 2-Methyl-4-(trifluormethyl)-1,3-thiazol-5-carbonylchlorid in 10 ml Acetonitril wird für 16 h bei Raumtemperatur gerührt. Zur Aufarbeitung wird die Base abfiltriert, das Filtrat eingeengt und mittels Säulenchromatographie gereinigt. Der Rückstand wird mit Cyclohexan/Essigsäureethylester (2:1) an Kieselgel chromatographiert.

Man erhält 15,7 g (83 % d. Th.) *N*-(2-Iodphenyl)-2-methyl-4-(trifluormethyl)-1,3-thiazol-5-carboxamid mit dem logP (pH2,3) = 2,81.

### Beispiel (IV-2)

Analog Beispiel (IV-1) wurde auch *N*-(2-Bromphenyl)-2-methyl-4-(trifluormethyl)-1,3-thiazol-5-carboxamid mit dem logP (pH2,3) = 2,79 erhalten.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Sphaerotheca-Test (Gurke) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von ***Sphaerotheca fuliginea*** inokuliert. Die Pflanzen werden dann bei ca. 23°C und einer relativen Luftfeuchtigkeit von ca. 70% im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

| **Tabelle A** | | | |
|---|---|---|---|
| **Sphaerotheca-Test (Gurke) / protektiv** | | | |
| Bsp. | Wirkstoff | Aufwandmenge an Wirkstoff in g/ha | % Wirkungsgrad |
| Gemäß EP 0 545 099: | | | |
| 3.37 | 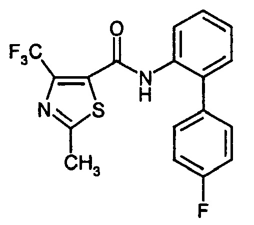 | 10 | 20 |
| | 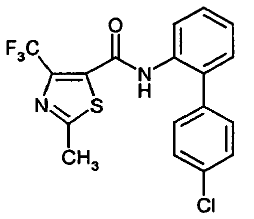 | 10 | 30 |

| Erfindungsgemäß: | | | |
|---|---|---|---|
| 1 | 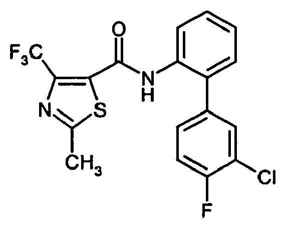 | 10 | 83 |
| 4 | 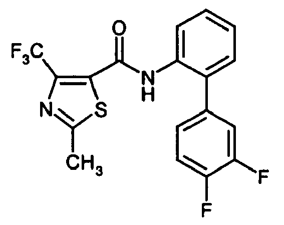 | 10 | 90 |
| 7 | 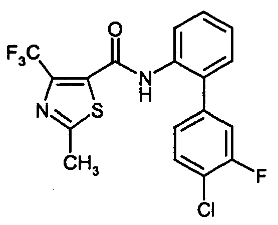 | 10 | 95 |
| 9 | 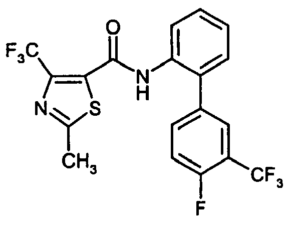 | 10 | 90 |
| 10 | 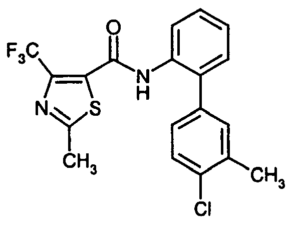 | 10 | 98 |
| 11 | 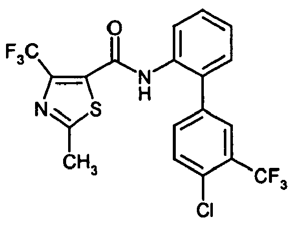 | 10 | 100 |
| 12 | 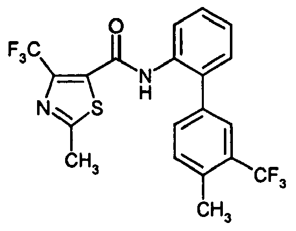 | 10 | 100 |
| 13 | 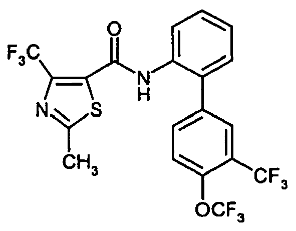 | 10 | 85 |
| 17 | 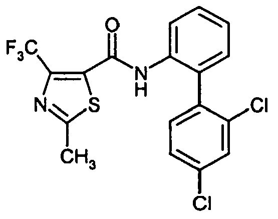 | 10 | 90 |
| 19 | 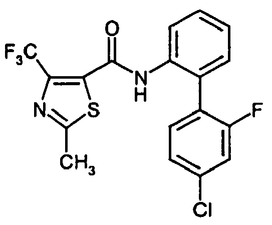 | 10 | 81 |

### Beispiel B

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

| **Tabelle B:** | | | |
|---|---|---|---|
| **Venturia - Test (Apfel) / protektiv** | | | |
| Bsp. | Wirkstoff | Aufwandmenge an Wirkstoff in g/ha | % Wirkungsgrad |
| Gemäß EP 0 545 099: | | | |
| 3.37 | 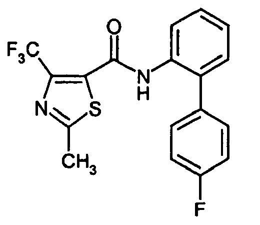 | 10 | 32 |
| | 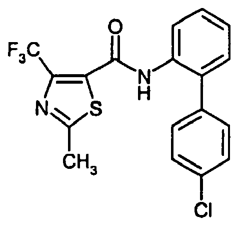 | 10 | 76 |

| Erfindungsgemäß: | | | |
|---|---|---|---|
| 1 | 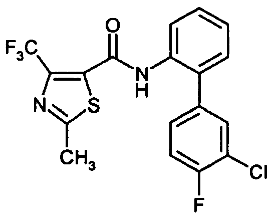 | 10 | 100 |
| 4 | 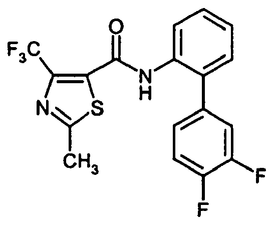 | 10 | 100 |
| 5 | 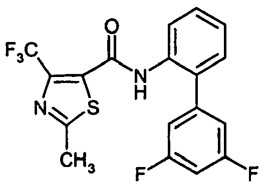 | 10 | 94 |
| 6 | 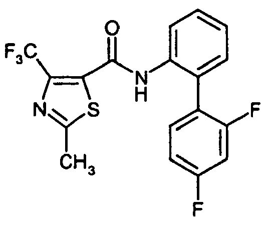 | 10 | 99 |
| 7 | 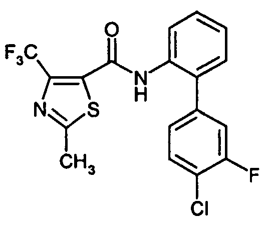 | 10 | 100 |
| 10 | 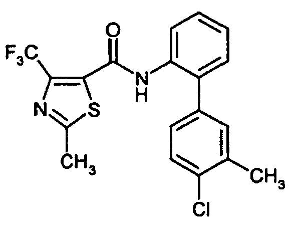 | 10 | 100 |
| 11 | 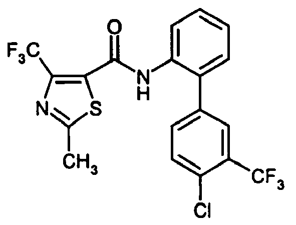 | 10 | 100 |
| 14 | 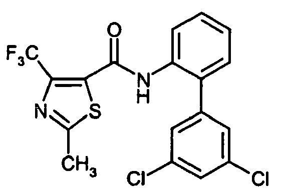 | 10 | 100 |
| 18 | 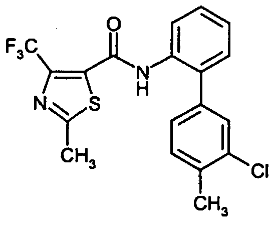 | 10 | 100 |
| 19 | 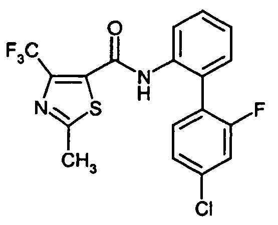 | 10 | 100 |

### Beispiel C

### Botrytis - Test (Bohne) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

| **Tabelle C** | | | |
|---|---|---|---|
| **Botrytis - Test (Bohne) / protektiv** | | | |
| Bsp. | Wirkstoff | Aufwandmenge an Wirkstoff in g/ha | Wirkungsgrad |
| Gemäß EP 0 545 099: | | | |
| 3.37 | 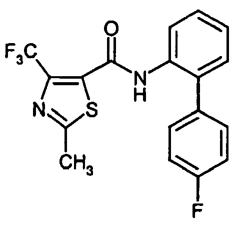 | 100 | 50 |
| | 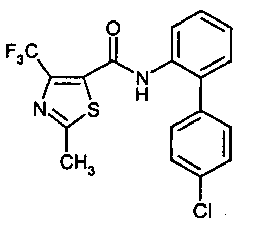 | 100 | 76 |

| Erfindungsgemäß: | | | |
|---|---|---|---|
| 1 | 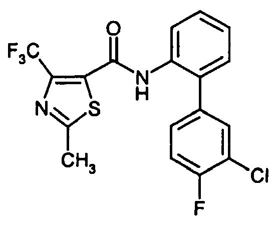 | 100 | 82 |
| 7 | 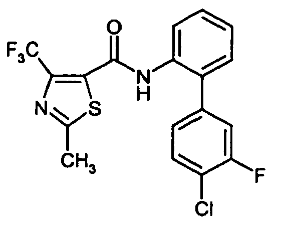 | 100 | 92 |
| 10 | 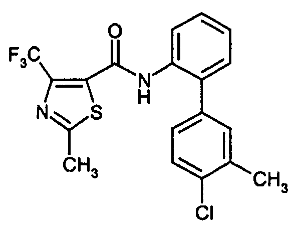 | 100 | 95 |
| 17 | 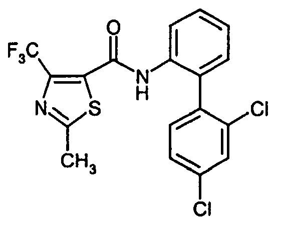 | 100 | 87 |
| 18 | 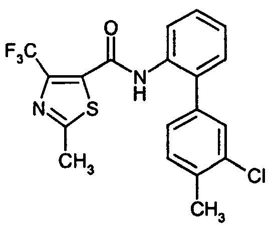 | 100 | 99 |
| 19 | 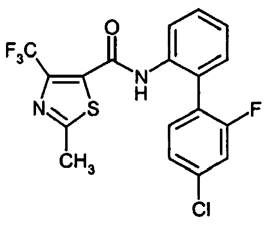 | 100 | 99 |

### Beispiel D

### In vitro-Test zur ED₅₀-Bestimmung bei Mikroorganismen

In die Kavitäten von Mikrotiterplatten wird eine methanolische Lösung des zu prüfenden Wirkstoffs, versetzt mit dem Emulgator PS 16, pipettiert. Nachdem das Lösungsmittel abgedampft ist, werden je Kavität 200 µl Potatoe-Dextrose-Medium hinzugefügt.

Das Medium wurde vorher mit einer geeigneten Konzentration von Sporen bzw. Mycel des zu prüfenden Pilzes versetzt.

Die resultierenden Konzentrationen des Wirkstoffs betragen 0.1, 1, 10 und 100 ppm. Die resultierende Konzentration des Emulgators beträgt 300 ppm.

Die Platten werden anschließend 3-5 Tage auf einem Schüttler bei einer Temperatur von 22°C inkubiert bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen wird die Wirkstoffdosis, die zu einer 50%igen Hemmung des Pilzwachstums gegenüber der unbehandelten Kontrolle führt (ED₅₀), berechnet.

| **Tabelle D:** | | | |
|---|---|---|---|
| **In vitro-Test zur ED₅₀-Bestimmung bei Mikroorganismen** | | | |
| Bsp. | Wirkstoff | Mikroorganismus | ED₅₀-Wert |
| Gemäß EP 0 545 099: | | | |
| 3.37 | 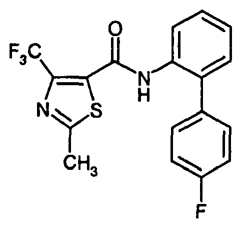 | Rhizoctonia solani | > 100 |
| | | Septoria tritici | 84,24 |
| | 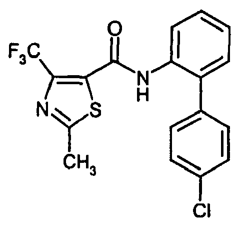 | Rhizoctonia solani | > 100 |
| | | Septoria tritici | > 100 |

| Erfindungsgemäß: | | | |
|---|---|---|---|
| 10 | 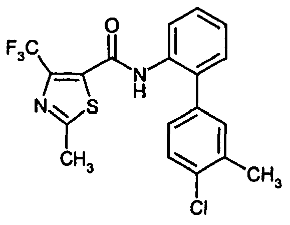 | Rhizoctonia solani | < 0,1 |
| | | Septoria tritici | < 0,1 |
| 11 | 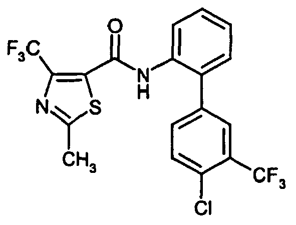 | Rhizoctonia solani | < 0,1 |
| | | Septoria tritici | 1,42 |
| 12 | 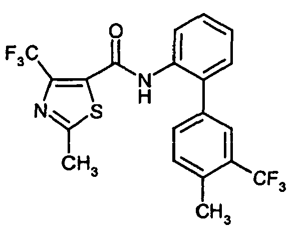 | Rhizoctonia solani | < 0,1 |
| | | Septoria tritici | 3,16 |
| 14 | 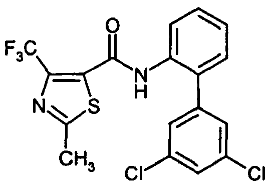 | Rhizoctonia solani | < 0,1 |
| | | Septoria tritici | 0,32 |
| 16 | 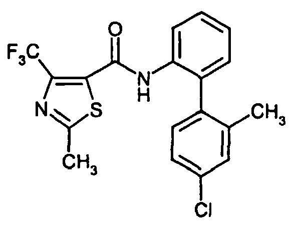 | Rhizoctonia solani | 0,45 |
| | | Septoria tritici | 0,89 |

## Patentansprüche

1. Thiazolylcarboxanilide der Formel (I) 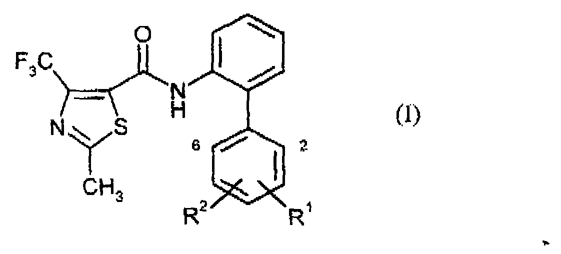 in welcher
R¹ und R² unabhängig voneinander für Halogen, Cyano, Nitro, C₁-C₆-Alkyl, C₂-C₆-Alkenyl, C₁-C₄-Alkoxy, C₁-C₄-Alkylthio, C₁-C₄-Alkylsulfonyl, C₃-C₆-Cycloalkyl, oder für C₁-C₄-Halogenalkyl, C₁-C₄-Halogenalkoxy, C₁-C₄-Halogenalkylthio oder C₁-C₄-Halogenalkylsulfonyl mit jeweils 1 bis 5 Halogenatomen stehen,
R¹ und R² außerdem, wenn sie in ortho-Position zueinander stehen, gemeinsam für gegebenenfalls durch Halogen oder C₁-C₆-Alkyl substituiertes Alkenylen stehen.

2. Thiazolylcarboxanilide der Formel (I) gemäß Anspruch 1, in welcher
R¹ und R² unabhängig voneinander für Fluor, Chlor, Brom, Cyano, Nitro, Methyl, Ethyl, n- oder i-Propyl, n-, i-, s- oder t-Butyl, Methoxy, Ethoxy, Methylthio, Ethylthio, n- oder i-Propylthio, Cyclopropyl, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio stehen,
R¹ und R² außerdem, wenn sie in ortho-Position zueinander stehen, gemeinsam für gegebenenfalls durch Fluor, Chlor, Brom oder Methyl substituiertes Butadienylen stehen.

3. Thiazolylcarboxanilide der Formel (1) gemäß Anspruch 1, in weicher
R¹ und R² unabhängig voneinander für Fluor, Chlor, Brom, Methyl, Trifluormethyl, Difluormethoxy oder Trifluormethoxy stehen.

4. Thiazolylcarboxanilide der Formel (I) gemäß Anspruch 1, in welcher R¹ für Fluor und R² für Chlor steht.

5. Thiazolylcarboxanilide der Formel (I) gemäß Anspruch 1, in welcher R¹ für Fluor und R² für Fluor steht.

6. Thiazolylcarboxanilide der Formel (I) gemäß Anspruch 1, in welcher R¹ für Methyl oder Trifluormethyl steht.

7. Verfahren zum Herstellen von Thiazolylcarboxaniliden der Formel (I) gemäß Anspruch 1, **dadurch gekennzeichnet, dass** man
a) Thiazolcarbonsäurehalogenide der Formel (II) 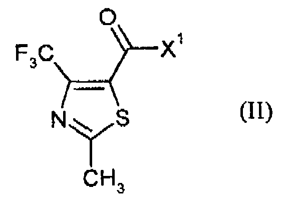 in welcher
X¹ für Halogen steht,
mit Anilinderivaten der Formel (III) 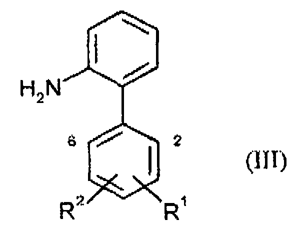 in welcher
R¹ und R² die in Anspruch 1 angegebenen Bedeutungen haben,
gegebenenfalls in Gegenwart eines Säurebindemittels und gegebenenfalls in Gegenwart eines Verdünnungsmittels umsetzt, oder
b) Halogenthiazolcarboxanilide der Formel (IV) 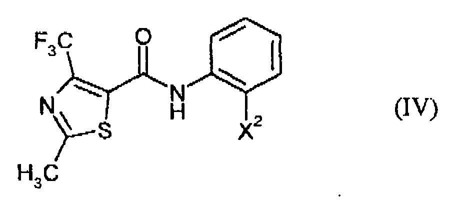 in welcher
X² für Brom oder lod steht,
mit Boronsäurederivaten der Formel (V) 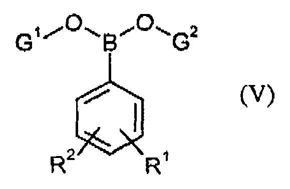 in welcher
R¹ und R² die in Anspruch 1 angegebenen Bedeutungen haben,
G¹ und G² jeweils für Wasserstoff oder zusammen für Tetramethylethylen stehen,
in Gegenwart eines Katalysators, gegebenenfalls in Gegenwart eines Säurebindemittels und gegebenenfalls in Gegenwart eines Verdünnungsmittels umsetzt, oder
c) Thiazolcarboxanilid-Boronsäure-Derivate der Formel (VI) 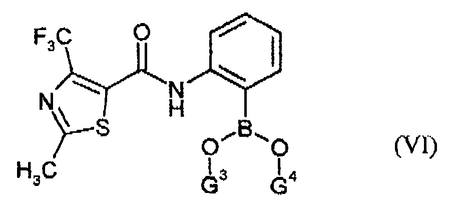 in welcher
G³ und G⁴ jeweils für Wasserstoff oder zusammen für Tetramethylethylen stehen,
mit Halogenbenzolderivaten der Formel (VII) 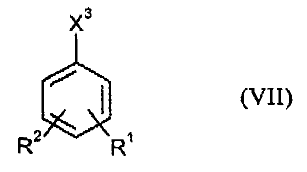 in welcher
R¹ und R² die in Anspruch 1 angegebenen Bedeutungen haben und
X³ für Brom, Iod oder Trifluonnethylsulfonyloxy steht,
in Gegenwart eines Katalysators, gegebenenfalls in Gegenwart eines Säurebindemittels und gegebenenfalls in Gegenwart eines Verdünnungsmittels umsetzt.

8. Mittel zum Bekämpfen unerwünschter Mikroorganismen, **gekennzeichnet durch** einen Gehalt an mindestens einem Thiazolylcarboxanilid der Formel (I) gemäß Anspruch 1 neben Streckmitteln und/oder oberflächenaktiven Stoffen.

9. Verwendung von Thiazolylcarboxaniliden der Formel (I) gemäß Anspruch 1 zum Bekämpfen unerwünschter Mikroorganismen im Pflanzenschutz oder im Materialschutz.

10. Verfahren zum Bekämpfen unerwünschter Mikroorganismen im Pflanzenschutz oder im Materialschutz, **dadurch gekennzeichnet, dass** man Thiazolylcarboxanilide der Formel (1) gemäß Anspruch 1 auf die Mikroorganismen und/oder deren Lebensraum ausbringt.

11. Verfahren zum Herstellen von Mitteln zum Bekämpfen unerwünschter Mikroorganismen, **dadurch gekennzeichnet, dass** man, Thiaxolylcarboxanilide der Formel (I) gemäß Anspruch 1 mit Streckmitteln und/oder oberflächenaktiven Stoffen vermischt.

## Claims

1. Thiazolylcarboxanilides of the formula (I) 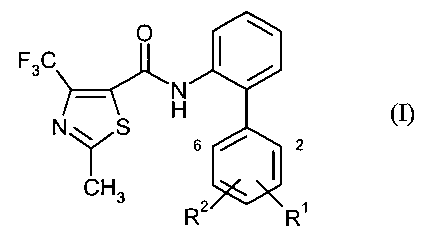 in which
R¹ and R² independently of one another represent halogen, cyano, nitro, C₁-C₆-alkyl, C₂-C₆-alkenyl, C₁-C₄-alkoxy, C₁-C₄-alkylthio, C₁-C₄-alkylsulphonyl, C₃-C₆-cycloalkyl, or represent C₁-C₄-haloalkyl, C₁-C₄-haloalkoxy, C₁-C₄-haloalkylthio or C₁-C₄-haloalkylsulphonyl having in each case 1 to 5 halogen atoms,
R¹ and R² furthermore, if they are arranged ortho to one another, together represent optionally halogen- or C₁-C₆-alkyl-substituted alkenylene.

2. Thiazolylcarboxanilides of the formula (I) according to Claim 1 in which
R¹ and R² independently of one another represent fluorine, chlorine, bromine, cyano, nitro, methyl, ethyl, n- or i-propyl, n-, i-, s- or t-butyl, methoxy, ethoxy, methylthio, ethylthio, n- or i-propylthio, cyclopropyl, trifluoromethyl, trichloromethyl, trifluoroethyl, difluoromethoxy, trifluoromethoxy, difluorochloromethoxy, trifluoroethoxy, difluoromethylthio, difluorochloromethylthio or trifluoromethylthio,
R¹ and R² furthermore, if they are arranged ortho to one another, together represent optionally fluorine-, chlorine-, bromine- or methyl-substituted butadienylene.

3. Thiazolylcarboxanilides of the formula (I) according to Claim 1 in which
R¹ and R² independently of one another represent fluorine, chlorine, bromine, methyl, trifluoromethyl, difluoromethoxy or trifluoromethoxy.

4. Thiazolylcarboxanilides of the formula (I) according to Claim 1 in which R¹ represents fluorine and R² represents chlorine.

5. Thiazolylcarboxanilides of the formula (I) according to Claim 1 in which R¹ represents fluorine and R² represents fluorine.

6. Thiazolylcarboxanilides of the formula (I) according to Claim 1 in which R¹ represents methyl or trifluoromethyl.

7. Process for preparing thiazolylcarboxanilides of the formula (I) according to Claim 1, **characterized in that**
a) thiazolcarbonyl halides of the formula (II) 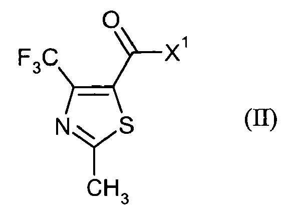 in which
X¹ represents halogen,
are reacted with aniline derivatives of the formula (III) 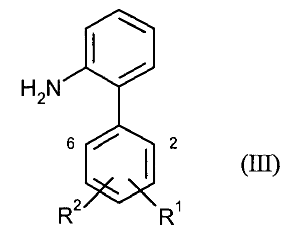 in which
R¹ and R² are as defined in Claim 1,
if appropriate in the presence of an acid binder and if appropriate in the presence of a diluent, or
b) halothiazolcarboxanilides of the formula (IV) 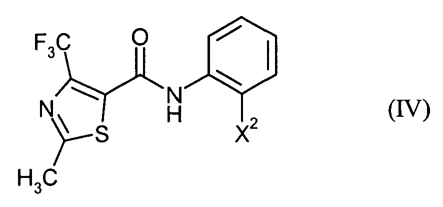 in which
X² represents bromine or iodine,
are reacted with boronic acid derivatives of the formula (V) 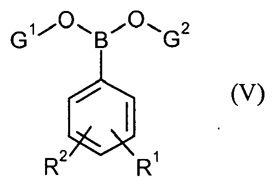 in which
R¹ and R² are as defined in Claim 1,
G¹ and G² each represent hydrogen or together represent tetramethylethylene,
in the presence of a catalyst, if appropriate in the presence of an acid binder and if appropriate in the presence of a diluent, or
c) Thiazolcarboxanilide boronic acid derivatives of the formula (VI) 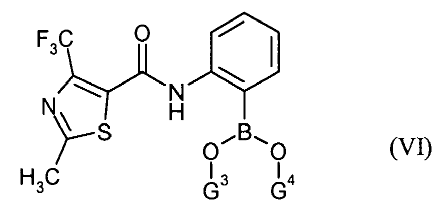 in which
G³ and G⁴ each represent hydrogen or together represent tetramethylethylene,
are reacted with halobenzene derivatives of the formula (VII) 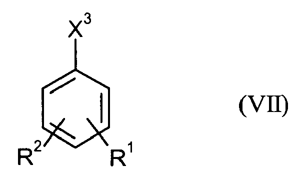 in which
R¹ and R² are as defined in Claim 1 and
X³ represents bromine, iodine or trifluoromethylsulphonyloxy
in the presence of a catalyst, if appropriate in the presence of an acid binder and if appropriate in the presence of a diluent.

8. Composition for controlling unwanted microorganisms, **characterized in that** it comprises at least one thiazolylcarboxanilide of the formula (I) according to Claim 1, in addition to extenders and/or surfactants.

9. Use of thiazolylcarboxanilides of the formula (I) according to Claim 1 for controlling unwanted microorganisms in crop protection or in the protection of materials.

10. Method for controlling unwanted microorganisms in crop protection or in the protection of materials, **characterized in that** thiazolylcarboxanilides of the formula (I) according to Claim 1 are applied to the microorganisms and/or their habitat.

11. Process for preparing compositions for controlling unwanted microorganisms, **characterized in that** thiazolylcarboxanilides of the formula (I) according to Claim 1 are mixed with extenders and/or surfactants.

## Revendications

1. Thiazolylcarboxanilides de formule (I) 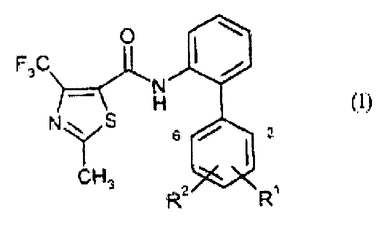 dans laquelle
R¹ et R² représentent, indépendamment l'un de l'autre, un halogène, un groupe cyano, nitro, un reste alkyle en C₁ à C₆, alcényle en C₂ à C₆, alkoxy en C₁ à C₄, alkylthio en C₁ à C₄, alkylsulfonyle en C₁ à C₄, cycloalkyle en C₃ à C₆ ou un reste halogénalkyle en C₁ à C₄, halogénalkoxy en C₁ à C₄, halogénalkylthio en C₁ à C₄ ou halogénalkylsulfonyle en C₁ à C₄ ayant chacun 1 à 5 atomes d'halogène,
R¹ et R² forment en outre un groupe alcénylène éventuellement substitué par un halogène ou par un radical alkyle en C₁ à C₆, lorsqu'ils sont en position ortho l'un par rapport à l'autre.

2. Thiazolylcarboxanilides de formule (I) suivant la revendication 1, formule dans laquelle
R¹ et R² représentent, indépendamment l'un de l'autre, le fluor, le chlore, le brome, un groupe cyano, nitro, un reste méthyle, éthyle, n-propyle, isopropyle, n-butyle, isobutyle, sec.-butyle, tertiobutyle, méthoxy, éthoxy, méthylthio, éthylthio, n-propylthio, isopropylthio, cyclopropyle, trifluorométhyle, trichlorométhyle, trifluoréthyle, difluorométhoxy, trifluorométhoxy, difluorochlorométhoxy, trifluoréthoxy, difluorométhylthio, difluorochlorométhylthio ou trifluorométhylthio,
R¹ et R² forment en outre un groupe butadiénylène éventuellement substitué par du fluor, du chlore, du brome ou un radical méthyle, lorsqu'ils sont en position ortho l'un par rapport à l'autre.

3. Thiazolylcarboxanilides de formule (I) suivant la revendication 1, formule dans laquelle
R¹ et R² représentent, indépendamment l'un de l'autre, le fluor, le chlore, le brome, un reste méthyle, trifluorométhyle, difluorométhoxy ou trifluorométhoxy.

4. Thiazolylcarboxanilides de formule (I) suivant la revendication 1, formule dans laquelle R¹ représente le fluor et R² représente le chlore.

5. Thiazolylcarboxanilides de formule (I) suivant la revendication 1, formule dans laquelle R¹ représente le fluor et R² représente le fluor.

6. Thiazolylcarboxanilides de formule (I) suivant la revendication 1, formule dans laquelle R¹ est un reste méthyle ou trifluorométhyle.

7. Procédé de production de thiazolylcarboxanilides de formule (I) suivant la revendication 1, **caractérisé en ce que**:
a) on fait réagir des halogénures d'acide thiazolcarboxylique de formule (II) 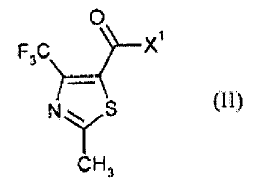 dans laquelle
X¹ représente un halogène
avec des dérivés d'aniline de formule (III) 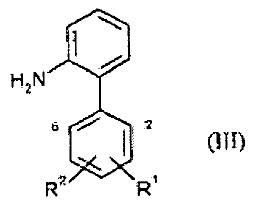 dans laquelle
R¹ et R² ont les définitions indiquées dans la revendication 1, éventuellement en présence d'un accepteur d'acide et, le cas échéant, en présence d'un diluant, ou bien
b) on fait réagir des halogénothiazolcarboxanilides de formule (IV) 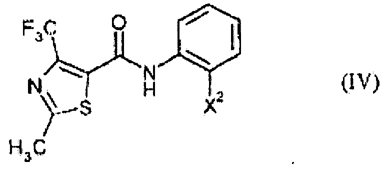 dans laquelle
X² représente le brome ou l'iode,
avec des dérivés d'acides boroniques de formule (V) 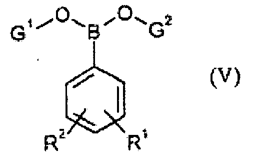 dans laquelle
R¹ et R² ont les définitions indiquées dans la revendication 1,
G¹ et G² représentent chacun l'hydrogène ou forment ensemble un groupe tétraméthyléthylène,
en présence d'un catalyseur, éventuellement en présence d'un accepteur d'acide et, le cas échéant, en présence d'un diluant, ou bien
c) on fait réagir des dérivés d'acides boroniques de thiazolcarboxanilide de formule (VI) 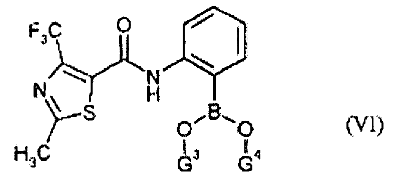 dans laquelle
G³ et G⁴ représentent chacun l'hydrogène ou forment ensemble un groupe tétraméthyléthylène,
avec des dérivés benzéniques halogénés de formule (VII) 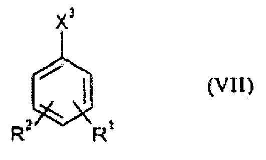 dans laquelle
R¹ et R² ont les définitions indiquées dans la revendication 1, et
X³ représente le chrome, l'iode ou un groupe trifluorométhylsulfonyloxy,
en présence d'un catalyseur, éventuellement en présence d'un accepteur d'acide et, le cas échéant, en présence d'un diluant.

8. Composition destinée à combattre des micro-organismes indésirables, **caractérisée par** un teneur en au moins un thiazolylcarboxanilide de formule (I) suivant la revendication 1, à côté de diluants et/ou d'agents tensioactifs.

9. Utilisation de thiazolylcarboxanilides de formule (I) suivant la revendication 1 pour combattre des micro-organismes indésirables dans la protection des plantes ou dans la protection des matériaux.

10. Procédé pour combattre des micro-organismes indésirables dans la protection des plantes ou dans la protection des matériaux, **caractérisé en ce qu'**on applique des thiazolylcarboxanilides de formule (I) suivant la revendication 1 sur les micro-organismes et/ou sur leur milieu.

11. Procédé de préparation de compositions destinées à combattre des micro-organismes indésirables, **caractérisé en ce qu'**on mélange des thiazolylcarboxanilides de formule (I) suivant la revendication 1 avec des diluants et/ou avec des agents tensioactifs.
